# EUROPEAN PATENT APPLICATION

(11) **EP 3 945 094 A1**
(43) Date of publication of application: **02.02.2022**
(21) Application number: 20188851.8
(22) Date of filing: 31.07.2020
(51) Int. Cl.: C07K 14/005, C12N 7/00

(54) **REPLICATION-DEFICIENT ADENOVIRUS**

(71) Applicant: Heinrich-Pette-Institut, 20251 Hamburg (DE)
(72) Inventor: BODDIN, Jana, 23843 Bad Oldesloe (DE); DOBNER, Thomas, 20251 Hamburg (DE)
(74) Representative: Uexküll & Stolberg

(57) **Abstract**

The present invention generally relates to the field of adenoviruses and adenoviral vectors that can be used as vaccines and gene therapy vectors. More specifically, the present invention relates to an adenovirus or an adenoviral vector that comprises a mutated DNA-binding protein that inhibits adenoviral DNA replication in a cell infected with a virus expressing said protein. The invention further relates to a nucleotide sequence encoding the mutated DNA-binding protein. In another aspect, the invention provides pharmaceutical compositions, vaccines and cells that comprise the mutated protein, a nucleotide sequence encoding same, or a modified adenovirus or adenoviral vector comprising any of those. The invention also relates to the use of the mutated protein, a nucleotide sequence encoding the same, or an adenovirus or recombinant adenoviral vector comprising any of those for the preparation of a vaccine.

## Description

The present invention generally relates to the field of adenoviruses and adenoviral vectors that can be used as vaccines and gene therapy vectors. More specifically, the present invention relates to an adenovirus or an adenoviral vector that comprises a mutated DNA-binding protein that inhibits adenoviral DNA replication in a cell infected with a virus expressing said protein. The invention further relates to a nucleotide sequence encoding the mutated DNA-binding protein. In another aspect, the invention provides pharmaceutical compositions, vaccines and cells that comprise the mutated protein, a nucleotide sequence encoding same, or a modified adenovirus or adenoviral vector comprising any of those. The invention also relates to the use of the mutated protein, a nucleotide sequence encoding same, or an adenovirus or recombinant adenoviral vector comprising any of those for the preparation of a vaccine.

### BACKGROUND OF THE INVENTION

Adenoviruses belong to the virus family Adenoviridae. Adenoviruses are non-enveloped double-stranded DNA viruses with a diameter of 90-100 nm. They consist of a protein capsid that contains group and type-specific antigens. Adenoviruses are highly resistant to environmental influences and may remain infectious for weeks at room temperature.

The family Adenoviridae is divided into 5 genera, depending on host specificity. A distinction is made between adenoviruses of mammals (Mastadenoviridae), birds (Aviadenoviridae), reptiles (Atadenoviridae), amphibians (Siadenoviridae) and fish (Ichtadenoviridae). Recently a sixth genus, Testadenoviridae, has been proposed for adenoviruses of turtles. The genus Mastadenoviridae includes human adenoviruses (HAdV) with more than 100 types, which are currently divided into 7 serologically distinguishable species (A-G). The types were distinguished by their genome sequence, oncogenicity in immunosuppressed rodents and haemagglutination properties.

Adenoviruses spread globally with a high prevalence and mostly lead to an infection already in childhood (Mitchell et al., 2000; Ampuero et al., 2012; Lin et al., 2004; Mahy & van Regenmortel, 2010). Adenoviral infections are usually asymptomatic, but can also cause serious diseases. In most cases these viruses cause ocular, respiratory or gastrointestinal infections. Less common are urinary tract infections, hepatitis and meningoencephalitis. Typical diseases that are caused by human pathogenic adenoviruses include keratoconjunctivitis epidemica (adenovirus types 8, 19, 37), acute respiratory diseases (types 1-3, 4, 6, 7, 14, 21), pharyngoconjunctival fever (types 3, 7, 14), follicular conjunctivitis (types 3, 4, 7), gastroenteritis (types 40, 41, 31), gastroenteritis with mesenteric lymphadenopathy (types 1, 2, 5, 6), pneumonia (types 1-4, 7), and acute febrile pharyngitis (types 1-3, 5-7).

It is common for adenovirus infections to occur in large numbers, particularly in community facilities. Cases reported and confirmed by laboratory diagnostics reflect only a fraction of the actual morbidity, because the diagnosis is often only made clinically. Especially in immunocompromised patients, like recipients of hematopoietic stem cells or organ transplants, adenoviruses may cause severe infections with frequently fatal consequences (Lion et al., 2010; Abe et al., 2003; Kolawole et al., 2014; Carrigan, 1997). Also, an extensive recombination of adenovirus has been observed in immunocompromised patients, which further increases the risk of severe infections in these patients.

To date, there is no effective antiviral therapy or a cross-species vaccine that can prevent severe courses of adenoviral infections. Accordingly, there is a need for attenuated adenoviruses that can be used for developing effective vaccines that protect against adenoviral infections. Preferably, the attenuated adenoviruses should be easy to prepare and provide a high safety level that allows their use in humans. Such attenuated adenoviruses could also be used as viral vectors in gene therapy processes for introducing a nucleic acid, such as a transgene, into a subject.

### DESCRIPTION OF THE INVENTION

It has now been surprisingly found that a mutation in a defined sequence motif of 4 amino acids within the adenoviral DNA-binding protein (DBP) completely blocks the ability of a virus expressing such protein to replicate its DNA in the cell after infection. Accordingly, an adenovirus that has been modified by inclusion of a mutation into the sequence motif within the adenoviral DBP is unable to replicate its genome and, as a consequence, to produce virus progeny. Accordingly, such modified adenovirus is highly suitable for being used in therapeutic approaches, in particular in therapeutic approaches treating humans.

The adenoviral DBP, a product of the E2A gene (early region 2A), is well known and has been reported to be expressed early as well as late in infection (Chow et al., 1980; Flint & Sharp, 1976; Voelkerding & Klessig, 1986). DBP is able to bind RNA, dsDNA and ssDNA, whereby latter is completely coated with the protein to protect the DNA from degradation by nucleases (van der Vliet & Levine, 1973; Monaghan et al., 1994). The protein promotes the viral DNA replication by unwinding dsDNA through multimerization of the protein (Zijderveld & van der Vliet, 1994; Monaghan et al., 1994; Dekker et al., 1997). Further, DBP is involved in the recruitment of the pTP-pol-complex to the replication origin and stimulates the DNA binding of the viral polymerase by changing the DNA conformation (van Breukelen et al., 2000; Lindenbaum et al., 1986; van Breukelen et al., 2003). At early time points of infection, the protein localizes diffusely in the nucleus, during the late phase it accumulates in intranuclear, circular structures (Chow et al., 1980; Flint & Sharp, 1976; Voelkerding & Klessig, 1986; Pombo et al., 1994). These structures provide a hub for several viral and cellular proteins and correspond to viral replication centers, which are the hot spots for viral DNA replication, late gene expression and likely also for the assembly of new virus particles (Pombo et al., 1994; Hidalgo et al., 2016; Condezo & San Martin, 2017).

A colocalization of DBP and the cellular ubiquitin-specific protease 7 (USP7/HAUSP) has been described in the prior art. Specifically, it was observed that USP7 localizes in the replication centers after infection (Ching et al., 2013). USP7 plays important roles in various cellular processes including cell division, apoptosis, tumorigenesis and epigenetic regulation (Li et al., 2002; Song et al., 2008; van der Horst et al., 2006; Faustrup et al., 2009; Li et al., 2004; Khoronenkova & Dianov, 2013; Dar et al., 2013; Felle et al., 2011). Further, it has been shown that USP7 is involved in the infection progress of human immunodeficiency virus (HIV-1), Epstein-Barr virus (EBV), Kaposi sarcoma-associated herpesvirus (KSHV), herpes simplex virus type 1 (HSV-1) and cytomegalovirus (CMV) (Holowaty et al., 2003; Jäger et al., 2012; Canning et al., 2004; Salsman et al., 2012; Ali et al., 2017). In the specific context of adenoviral infection, USP7 seems to act as a proviral factor, since its inhibition by the use of HBX41108 or a knock-down of the protein leads to reduced viral replication and decreased protein levels of the viral early protein E1B-55K (Ching et al., 2013). Although USP7 and E1B-55K interact, the relocalization of USP7 into viral replication centers was also observed after infection with an E1B-55K-deleted mutant virus (Ching et al., 2013).

To clarify the relationship between DBP and USP7, DBP variants were generated, as explained in the below examples. Two putative USP7-binding motifs (UBM) in the amino acid sequence of DBP were selected for mutation, namely 72-Pro-Ser-Thr-Ser-77 and 350-Ser-Gly-Lys-Ser-355. The last serine residue of each motif was substituted from Ser to Ala to provide protein variants comprising the mutated motif 72-Pro-Ser-Thr-Ala-77 and 350-Ser-Gly-Lys-Ala-355, respectively. The resulting DBP variants were designated as DBP-S76A and DBP-S354A. The expression level of DBP-S76A was found to be comparable with wild-type DBP, whereas the expression level of DBP-S354A was found to be reduced.

The protein variants were tested in immunoprecipitation and GST pull-down analyses. Interaction of DBP-S354A with USP7 was found to be decreased in immunoprecipitation. Strikingly, the interaction of DBP-S76A with USP7 was completely abolished in immunoprecipitation and pull-down analysis. To clarify the functionality of the modified DBP during infection, virus mutants were generated that express the DBP variants. The virus mutant expressing DBP-S76A was designated UBM2, and the virus mutant expressing DBP-S354A was designated UBM5. The USP7-binding of the newly generated DBP-mutants was investigated in infection via immunoprecipitation analyses. It was found that DBP-S76A of UBM2 did not bind to USP7 in infection. Despite of decreased protein levels of DBP-S354A, the levels of coprecipitated USP7 with the UBM5-DBP are comparable to the wild-type virus, DBP-wt. Progeny production of infected H1299 cells with wild-type virus, UBM2 or UBM5 was determined by titration analyses and fluorescence focus identification assay, and it could be demonstrated that the amino acid exchange S76A in DBP has a mild effect on viral progeny production, while the amino acid exchange S354A in DBP completely prevents virus progeny production as a result of defective DNA replication.

The motif NH₂-Ser-[Gly/Ser/Ala]-[Lys/Arg]-Ser-COOH occurs in all HAdV types. The motif consists of 4 amino acids with a Ser residue both at the N-terminus and the C-terminus. The two amino acids flanked by the Ser residue are more variable. For example, the amino acid in position 2 of the motif can be Gly, Ser, or Ala. Similarly, the amino acid in position 3 of the motif can be either Lys or Arg. The motif can occur at slightly different positions of the DBP protein, depending on the length of the DBP in the respective HAdV type. However, based on the common knowledge and the instant disclosure, a skilled person would be readily able to identify the motif within the respective DBP. For example, in HAdV-A type 12, the motif occurs as NH₂-Ser-Gly-Lys-Ser-COOH at positions 306-309 of the full-length protein. In HAdV-B types 7, 16, and 68, the motif occurs as NH₂-Ser-Ser-Lys-Ser-COOH at positions 336-339 of the full-length protein. In HAdV-B types 11 and 35, the motif occurs as NH₂-Ser-Ser-Arg-Ser-COOH at positions 337-340 of the full-length protein. In HAdV-C types 1, 2 and 5, the motif occurs as NH₂-Ser-Gly-Lys-Ser-COOH at positions 351-354 of the full-length protein. In HAdV-D type 17, the motif occurs as NH₂-Ser-Gly-Lys-Ser-COOH at positions 309-312 of the full-length protein. In HAdV-E type 4, the motif occurs as NH₂-Ser-Ser-Lys-Ser-COOH at positions 330-333 of the full-length protein. In HAdV-F type 40, the motif occurs as NH₂-Ser-Ala-Lys-Ser-COOH at positions 297-300 of the full-length protein. In HAdV-F type 41, the motif occurs as NH₂-Ser-Ser-Lys-Ser-COOH at positions 298-301 of the full-length protein.

Therefore, in a first aspect the present invention refers to an adenoviral DBP comprising a mutation in the sequence motif NH₂-Ser-[Gly/Ser/Ala]-[Lys/Arg]-Ser-COOH, which inhibits adenoviral DNA replication in a cell infected with a virus expressing said protein. The mutation can be either a deletion of one or more amino acids in the above motif, an insertion of one or more amino acids, which disrupts the motif, or a substitution of one or more amino acids of the above motif. The ability of inhibiting DNA replication of an adenovirus mutant can be assessed as described in the example, e.g. by real-time PCR methods.

In one embodiment, the sequence motif NH₂-Ser-[Gly/Ser/Ala]-[Lys/Arg]-Ser-COOH in the DBP is disrupted by the deletion of one of the amino acids that contribute to the motif. For example, the Ser residue at the N-terminus of the motif can be deleted such that the motif NH₂-Ser-[Gly/Ser/Ala]-[Lys/Arg]-Ser-COOH is modified into NH₂-[Gly/Ser/Ala]-[Lys/Arg]-Ser-COOH. Similarly, the Ser residue at the C-terminus of the motif can be deleted, which means that the motif NH₂-Ser-[Gly/Ser/Ala]-[Lys/Arg]-Ser-COOH is modified into NH₂-Ser-[Gly/Ser/Ala]-[Lys/Arg]-COOH. In another embodiment, both Ser residues of the motif are deleted. In another embodiment, the Gly/Ser/Ala residue of the motif is deleted such that the motif NH₂-Ser-[Gly/Ser/Ala]-[Lys/Arg]-Ser-COOH is modified into NH₂-Ser-[Lys/Arg]-Ser-COOH. In yet another embodiment, the Lys/Arg residue of the motif is deleted such that the motif NH₂-Ser-[Gly/Ser/Ala]-[Lys/Arg]-Ser-COOH is modified into NH₂-Ser-[Gly/Ser/Ala]-Ser-COOH. Of course, it is also possible to delete the complete motif, i.e. all of the four amino acids.

Another option for incorporating a mutation into the sequence motif NH₂-Ser-[Gly/Ser/Ala]-[Lys/Arg]-Ser-COOH is to insert one or more amino acids. In this way, the motif is disrupted and can no longer contribute to the signaling interactions that normally provide for DNA replication. According to the invention, the one or more amino acids can be inserted in any position within the motif. For example, one or more amino acids, such as 2, 3, 4 or 5 amino acids, can be inserted between the Ser residue and the Gly/Ser/Ala residue. Alternatively, one or more amino acids, such as 2, 3, 4 or 5 amino acids, can be inserted between the Gly/Ser/Ala residue and the Lys/Arg residue. Likewise, one or more amino acids, such as 2, 3, 4 or 5 amino acids, can be inserted between the Lys/Arg residue and the C-terminally located Ser residue. It is of course also possible to insert amino acids in more than one position within the motif. Preferred insertions include, but are not restricted to
NH₂-Ser-Xₙ-[Gly/Ser/Ala]-[Lys/Arg]-Ser-COOH
NH₂-Ser-[Gly/Ser/Ala]-Xₙ-[Lys/Arg]-Ser-COOH
NH₂-Ser-[Gly/Ser/Ala]-[Lys/Arg]-Xₙ-Ser-COOH
NH₂-Ser-Xₙ-[Gly/Ser/Ala]-[Lys/Arg]-Xₙ-Ser-COOH
NH₂-Ser-Xₙ-[Gly/Ser/Ala]-Xₙ-[Lys/Arg]-Xₙ-Ser-COOH
wherein X is any amino acid and n preferably is an integer between 1 and 10, and more preferably between 1 and 5. It is particularly preferred that n corresponds to 1 or 2.

In yet another embodiment, the mutation within the sequence motif is an amino acid substitution. In principle, any of the four amino acid positions of the motif NH₂-Ser-[Gly/Ser/Ala]-[Lys/Arg]-Ser-COOH can be subjected to a substitution. It is preferred that the substitution is made at one of the Ser residues within the motif. It is particularly preferred that the substitution is made at the Ser residue located at the COOH terminus of the sequence motif. This serine residue is located at amino acid position 354 in the DBP of HAdV-C type 5 (HAdV-C5). If one or both of the Ser residues in the motif are substituted, it is preferred that the replacing amino acid is selected from the group of non-polar amino acids comprising glycine, valine, alanine, isoleucine, leucine, methionine, proline, phenylalanine, and tryptophan. A substitution of serine by alanine is particularly preferred. If the Gly/Ser/Ala residue is substituted, it is preferred that the replacing amino acid is selected from the group of polar amino acids comprising threonine, glutamine, asparagine, tyrosine, cysteine, histidine, arginine, lysine, aspartic acid and glutamic acid. If the Lys/Arg residue is substituted, it is preferred that the replacing amino acid is selected from the group of non-polar amino acids such as glycine, valine, alanine, isoleucine, leucine, methionine, proline, phenylalanine, and tryptophan. In general, any substitution within the motif NH₂-Ser-[Gly/Ser/Ala]-[Lys/Arg]-Ser-COOH is suitable, as long as it interferes with the ability of the resulting DBP variant to replicate the adenoviral DNA of a virus expressing the DBP variant. The ability of inhibiting DNA replication of an adenovirus mutant can be assessed as described in the example, e.g. by real-time PCR methods.

The mutation in the DBP protein inhibits adenoviral DNA replication in a cell, which has been infected with a virus type that expresses said mutated protein. Preferably, DNA replication is inhibited by at least 70%, more preferably at least 75%, at least 80%, at least 85%, at least 90%, at least 95% or more as compared to the corresponding wild-type adenovirus from which the mutated DBP is derived. For example, if the mutated DBP is derived from HAdV-C5, DNA replication will be inhibited in an adenovirus that has been modified to express the mutated DBP by at least 70%, more preferably at least 75%, at least 80%, at least 85%, at least 90%, at least 95% or more compared to wild-type HAdV-C5. In a particularly preferred embodiment, mutation in the DBP protein inhibits adenoviral DNA replication in a cell, which has been infected with a virus type that expresses said mutated protein by at least 98%, at least 99% or even completely. The inhibition of DNA replication can be determined by real time PCR approaches.

Any adenoviral DBP can be used for mutation in accordance with the present invention. However, it is preferred that the DBP is derived from an adenovirus of the genus Mastadenoviridae, i.e. from an adenovirus that occurs in mammals. It is even more preferred that the DBP is derived from a human adenovirus (HAdV). The group of HAdV presently comprises seven species (A-G) with more than 100 types. In a preferred embodiment, the adenoviral DBP used for mutation is derived from an HAdV type that is known to be pathogenic for humans, in particular an HAdV type selected from the group of types consisting of types 1, 2, 3, 4, 5, 6, 7, 31, 40, and 41.

In a preferred embodiment, the DBP, which is mutated is derived from the DBP of human adenovirus C type 5 (HAdV-C5). The amino acid sequences of the DBP of human HAdV-C5 is depicted in SEQ ID NO:1 herein. In this protein, the motif NH₂-Ser-Gly-Lys-Ser-COOH is located in amino acid positions 351-354. In the sequence set forth in SEQ ID NO:2, the motif has been altered to the motif NH₂-Ser-Gly-Lys-Ala-COOH. As shown in the examples below, this substitution of the C terminal Ser residue completely abrogates adenoviral DNA replication and results in an attenuated virus. Preferably, the mutated DBP comprises or consist of the sequence SEQ ID NO:2 or an amino acid sequence that comprises the altered motif NH₂-Ser-Gly-Lys-Ala-COOH and has at least 90% identity to the sequence SEQ ID NO:2.

Preferably, the DBP used for mutation according to the methods disclosed herein is derived from an adenovirus that is closely related to HAdV-C5 and therefore shares a particularly high degree of sequence identity with the amino acid sequence of SEQ ID NO:2. For example, the sequence identity is 91%, 92%, 93%, 94%, 95%, 96%, 97%, 98%, or 99%. Preferably, the sequence identity is determined over a length of at least 100 amino acids, more preferably at least 150 amino acids, at least 200 amino acids, at least 250 amino acids, at least 300 amino acids, at least 350 amino acids, at least 400 amino acids, at least 450 amino acids, at least 500 amino acids, or more.

In one embodiment, the DBP used for mutation shares a sequence identity with the amino acid sequence of SEQ ID NO:2 of at least 95% over a length of at least 200 amino acids, more preferably at least 300 amino acids, even more preferably at least 400 amino acids, even more preferably at least 450 amino acids, and even more preferably at least 500 amino acids. It is particularly preferred that the sequence identity is at least 95% over the full length of the protein. In another embodiment, the DBP used for mutation shares a sequence identity with the amino acid sequence of SEQ ID NO:2 of at least 96% over a length of at least 200 amino acids, more preferably at least 300 amino acids, even more preferably at least 400 amino acids, even more preferably at least 450 amino acids, and even more preferably at least 500 amino acids. It is particularly preferred that the sequence identity is at least 96% over the full length of the protein. In yet another embodiment, the DBP used for mutation shares a sequence identity with the amino acid sequence of SEQ ID NO:2 of at least 97% over a length of at least 200 amino acids, more preferably at least 300 amino acids, even more preferably at least 400 amino acids, even more preferably at least 450 amino acids, and even more preferably at least 500 amino acids. It is particularly preferred that the sequence identity is at least 97% over the full length of the protein. In yet another embodiment, the DBP used for mutation shares a sequence identity with the amino acid sequence of SEQ ID NO:2 of at least 98% over a length of at least 200 amino acids, more preferably at least 300 amino acids, even more preferably at least 400 amino acids, even more preferably at least 450 amino acids, and even more preferably at least 500 amino acids. It is particularly preferred that the sequence identity is at least 98% over the full length of the protein. In yet another embodiment, the DBP used for mutation shares a sequence identity with the amino acid sequence of SEQ ID NO:2 of at least 99% over a length of at least 200 amino acids, more preferably at least 300 amino acids, even more preferably at least 400 amino acids, even more preferably at least 450 amino acids, and even more preferably at least 500 amino acids. It is particularly preferred that the sequence identity is at least 99% over the full length of the protein.

In order to determine the sequence identity between two amino acid sequences, these sequences are usually aligned for optimal comparison. For example, gaps can be introduced in the sequence of a first amino acid sequence for optimal alignment with a second amino acid sequence. The amino acids at corresponding positions are then compared. If identical amino acids occur in corresponding positions in the first and second amino acid sequence, the sequences are identical at that position. A percentage sequence identity between two amino acid sequences means that, when aligned, the recited percentage of amino acids are identical in comparing both sequences. A percentage sequence identity can be determined by using software programs that are widely known in the art, for example the ALIGN program (version 2.0), which is part of the GCG sequence alignment software package. When utilizing the ALIGN program for comparing amino acid sequences, a PAM120 weight residue table, a gap length penalty of 12, and a gap penalty of 4 can be used.

The present invention also provides a nucleic acid encoding a mutated DBP protein as described hereinabove. A plasmid comprising such a nucleic acid is also provided. As used herein, a plasmid refers to an extrachromosomal circular DNA capable of autonomous replication in a cell.

In another aspect, the invention pertains to an adenovirus or a recombinant adenoviral vector that comprises a modified DBP as described herein or a nucleotide sequence encoding the same. As used herein, the term "adenovirus" refers to a virus or virus particle that can be categorized as an adenovirus, including all types and subtypes that occur naturally or have been recombinantly produced. In contrast, a "viral vector" refers to a virus or viral particle that comprises a polynucleotide, which is exogenous to the viral genome, such as a transgene, and which is to be delivered to a host cell by in vivo, ex vivo or in vitro methods. The adenovirus or adenoviral vector of the invention is preferably derived from an adenovirus of the genus Mastadenoviridae. More preferably, the adenovirus or adenoviral vector is or is derived from an HAdV, and more preferably from a type that is known to be pathogenic for humans. Most preferably, the adenovirus or adenoviral vector of the invention is or is derived from an HAdV type selected from types 1, 2, 3, 4, 5, 6, 7, 31, 40, and 41.

In a preferred embodiment, the adenovirus or a recombinant adenoviral vector of the invention comprises, as part of its genome, a nucleotide sequence encoding the modified DBP as defined above. It is particularly preferred that this adenovirus or a recombinant adenoviral vector only comprises a gene encoding the mutated DBP, but not any gene encoding the non-mutated version of the DBP. This ensures that the adenovirus or vector is unable to replicate its genome after infection of the target cell.

In yet another aspect, the invention relates to the use of a modified DBP as described herein, a nucleotide sequence or plasmid encoding the same, or an adenovirus or a recombinant adenoviral vector that comprises said modified DBP and/or nucleotide sequence in medicine, i.e. for therapeutic purposes. Since the mutated adenoviral DBP prevents viral DNA replication, the protein or a nucleotide sequence or plasmid encoding the same, as well as a virus or vector expressing said protein are useful for treating or preventing adenovirus infections. Accordingly, the invention particularly relates to a modified DBP as described herein, a nucleotide sequence or plasmid encoding the same or an adenovirus or a recombinant adenoviral vector that comprises said modified DBP or nucleotide sequence for use in a method of treating or preventing an adenovirus infection in a subject. The subject preferably is a mammalian subject, more preferably a human subject. The subject preferably suffers from infection with a HAdV type selected from types 1, 2, 3, 4, 5, 6, 7, 31, 40, and 41.

In yet another aspect, the invention refers to the mutated adenoviral DBP, a nucleotide sequence or plasmid encoding the same, or an adenovirus or a recombinant adenoviral vector that comprises said modified DBP and/or nucleotide sequence or plasmid for use in a method of vaccinating a subject. The subject preferably is a mammalian subject, more preferably a human subject. The vaccination is preferably carried out with a HAdV type, preferably a HAdV type selected from types 1, 2, 3, 4, 5, 6, 7, 31, 40, and 41. The vaccination with the adenovirus or recombinant adenoviral vector shall protect the subject against adenovirus infection, and preferably against a disease that is caused by an adenovirus and is selected from the group consisting of keratoconjunctivitis epidemica, acute respiratory diseases, pharyngoconjunctival fever, follicular conjuncttivitis, gastroenteritis, such as gastroenteritis with mesenteric lymphadenopathy, pneumonia, and pharyngitis.

The invention also provides a cell that comprises an adenoviral DBP as described herein, a nucleotide sequence or plasmid encoding the same, or an adenovirus or recombinant adenoviral vector that comprises said modified DBP and/or nucleotide sequence or plasmid. The cell can be any eukaryotic cell, but it will preferably be a mammalian cell, and more preferably a human cell. Preferably, the invention provides a cell that is transfected with an adenovirus or recombinant adenoviral vector which expresses the mutated adenoviral DBP of the invention.

In a further aspect, the invention provides a pharmaceutical composition or vaccine comprising a modified DBP as described herein, a nucleotide sequence or plasmid encoding the same or an adenovirus or a recombinant adenoviral vector that comprises said modified DBP and/or nucleotide sequence or plasmid. Preferably, the invention provides a pharmaceutical composition or vaccine comprising an adenovirus or an adenoviral vector of the present invention that expresses a mutated DBP as defined herein. The pharmaceutical composition can be formulated for various routes of administration. For example, the composition can be formulated for oral administration in the form of a capsule, a liquid or the like. However, it is preferred that the pharmaceutical composition or vaccine is administered parenterally, preferably by intravenous injection or intravenous infusion. The administration can be achieved, for example, by intravenous infusion, for example within 60 minutes, within 30 minutes or within 15 minutes. Compositions, which are suitable for administration by injection and/or infusion typically include solutions and dispersions, and powders from which corresponding solutions and dispersions can be prepared. Such compositions will comprise a mutated protein, nucleic acid, adenovirus or adenoviral vector as defined hereinabove and at least one pharmaceutically acceptable carrier. Suitable pharmaceutically acceptable carriers for intravenous administration include bacteriostatic water, Ringer's solution, physiological saline, phosphate buffered saline (PBS) and Cremophor EL^{™}. Sterile compositions for the injection and/or infusion can be prepared by introducing the mutated protein, nucleic acid, adenovirus or adenoviral vector as defined hereinabove in the required amount into an appropriate carrier, and then sterilizing by filtration. Compositions for administration by injection or infusion should remain stable under storage conditions after their preparation over an extended period of time. The compositions can contain a preservative for this purpose. Suitable preservatives include chlorobutanol, phenol, ascorbic acid and thimerosal. The preparation of corresponding formulations and suitable adjuvants is described, for example, in "Remington: The Science and Practice of Pharmacy," Lippincott Williams & Wilkins; 21st edition (2005).

The pharmaceutical composition will comprise the mutated DBP, the nucleotide sequence or plasmid encoding the same, or the adenovirus or adenoviral vector in a therapeutically effective amount, i.e., in an amount that is sufficient for improving at least one symptom of the disease to be treated to the patient or to prevent the progression of the disease to the patient. A therapeutically effective amount of the adenovirus or adenoviral vector causes a positive change in at least one of the symptoms, i.e., a change, which results in the phenotype of the affected subject approximating the phenotype of a healthy subject who does not suffer from the respective disease. In one preferred embodiment, the administration of the adenovirus or an adenoviral vector occurs in an amount, which leads to a complete or substantially complete healing of the disease or dysfunction to be treated. A therapeutically effective amount will generally be non-toxic for the subject who undergoes the treatment.

The exact amount of the protein, nucleotide sequence, plasmid, adenovirus or adenoviral vector, which must be administered to achieve a therapeutic effect depends on several parameters. Factors that are relevant to the amount of the adenovirus or adenoviral vector to be administered are, for example, the route of administration, the nature and severity of the disease, the disease history of the patient being treated, as well as the age, weight, height, and health of the patient. Furthermore, in gene therapy approaches, the expression level of the transgene, which is required to achieve a therapeutic effect, the immune response of the patient, as well as the stability of the gene product are relevant parameters for the amount to be administered. A therapeutically effective amount of the adenovirus or adenoviral vector can be determined by a person skilled in the art on the basis of general knowledge and the present disclosure.

If an adenovirus or adenoviral vector is used as a vaccine or therapeutic agent, the amount of said adenovirus or vector to be administered preferably corresponds to a dose in the range of 1.0 x 10¹⁰ to 1.0 x 10¹⁴ vg/kg (virus genomes per kg body weight), although a range of 1.0 x 10¹¹ to 1.0 x 10¹³ vg/kg is more preferred, and a range of 5.0 x 10¹¹ to 5.0 x 10¹² vg/kg is still more preferred, and a range of 1.0 x 10¹² to 5.0 x 10¹² is still more preferred. A dose of about 2.5 x 10¹² vg/kg is most preferred.

When formulated as a vaccine, the above components, e.g. the adenovirus or recombinant adenoviral vector, will be admixed with an adjuvant. An adjuvant is a compound that enhances the immune responses in a subject to whom the vaccine is administered. Adjuvants, which are commonly used for the preparation of vaccines include, but are not limited to, mineral salts, such as aluminium salts or calcium salts; oil-in-water emulsions; saponin compounds, such as QS7, QS17, QS18, or QS21; immunostimulatory oligonucleotides, such as oligonucleotides sequences containing a CpG motif; biodegradable microparticles, such as particles of poly-α-hydroxy acid, polyhydroxybutyric acid, polyorthoester, or the like; liposomes; muramyl peptides; and the like. According to a preferred embodiment of the present invention, the adjuvant used is an aluminium salt, in particular aluminium hydroxide.

In yet another aspect, the invention relates to the use of an adenoviral DBP as described herein, a nucleotide sequence or plasmid encoding the same, or an adenovirus or recombinant adenoviral vector as described herein for the preparation of a vaccine. This vaccine is preferably effective against a disease that is caused by adenovirus infection selected from the group consisting of keratoconjunctivitis epidemica, acute respiratory diseases, pharyngoconjunctival fever, follicular conjunctivitis, gastroenteritis, pneumonia, and pharyngitis.

The modified adenovirus or recombinant adenoviral vector of the invention is also useful in gene therapy approaches. Since an adenovirus or recombinant adenoviral vector that expresses a mutated DBP as defined hereinabove are unable to replicate after infection, they provide a high safety level when used as gene therapy vectors.

### BRIEF DESCRIPTION OF THE FIGURES

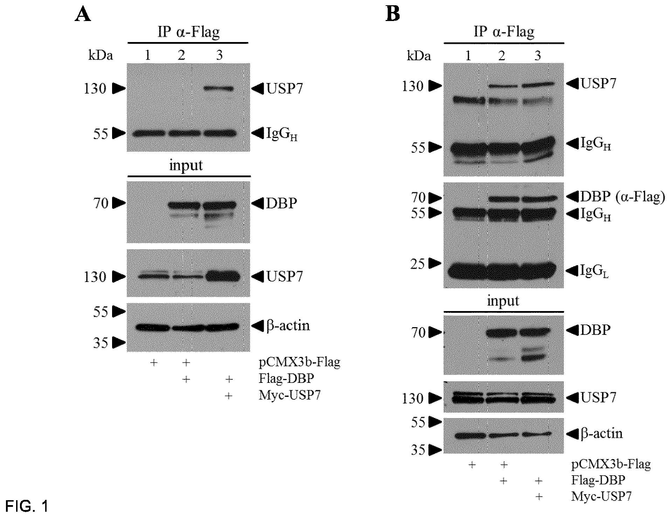

Figure 1 shows that DBP interacts with USP7 in transfected cells. H1299 cells (A) and HCT116 cells (B) were co-transfected with 10 µg of the plasmid constructs flag-DBP and myc-USP7. In order to transfect the cells with the same amount of plasmid-DNA between the different samples, the DNA amounts were adjusted with the expression vector pCMX3b-Flag. The cells were harvested 48 h p.t. (hours post transfection) and lysed. The flag-coupled DBP was immunoprecipitated using α-flag-antibody coupled agarose. The proteins were separated by SDS-PAGE and visualized by immunodetection. Precipitated and coprecipitated proteins were detected with the antibodies M2 (a-flag) and 3D8 (α-USP7).

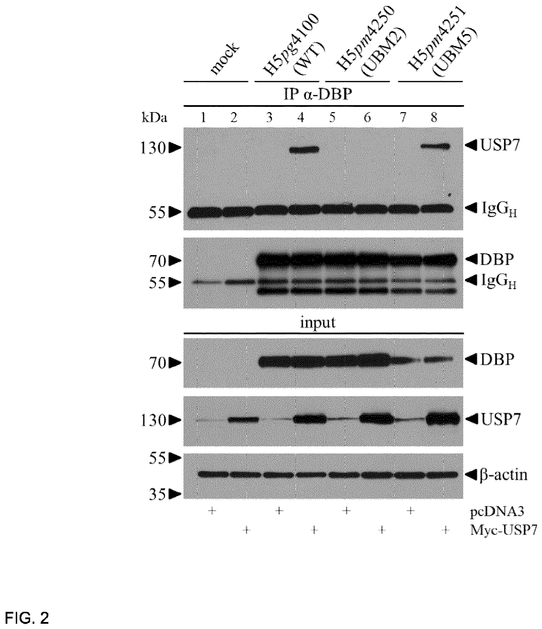

Figure 2 shows that DBP-S76A does not bind to USP7. H1299 cells were transfected with 10 µg of each of the plasmid constructs pcDNA3 or myc-USP7. 24 h after transfection the cells were infected with the viruses H5pg4100 (WT), H5pm4250 (UBM2) or H5pm4251 (UBM5) (m.o.i. 20), harvested 24 h.p.i/48 h p.t. and lysed. The DBP was immunoprecipitated using α-DBP-Ab coupled protein A-Sepharose, the proteins were separated by SDS-PAGE and visualized by immunodetection. Equilibrium amounts of specific proteins were detected with the antibodies B6-8 (a-DBP), 3D8 (α-USP7) and α-β-actin. Precipitated and coprecipitated protein (IP) was detected with the antibodies B6-8 (a-DBP) and 3D8 (α-USP7). The molecular weight in kDa is indicated on the left and the detected proteins on the right side of the figure.

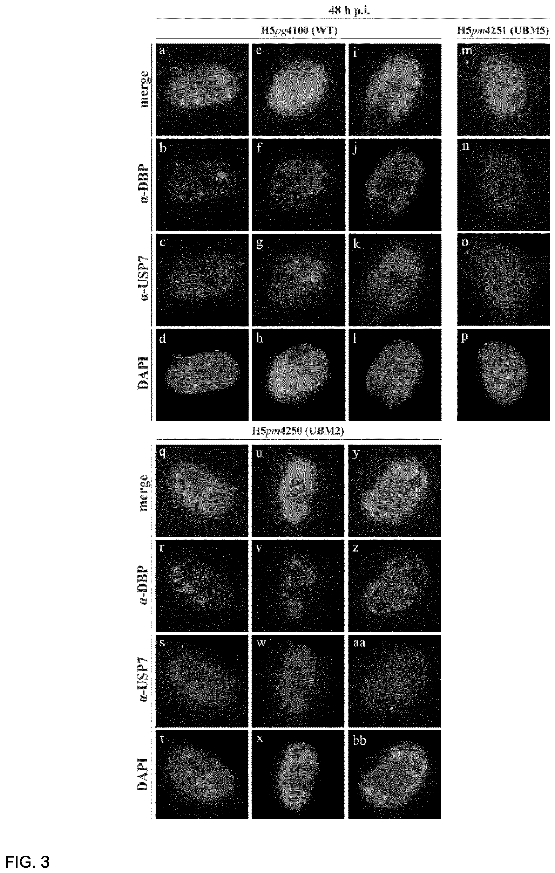

Figure 3 shows the localization of USP7 and DBP in H5pg4100 (WT), H5pm4250 (UBM2) or H5pm4251 (UBM5) infected HCT116 cells. HCT116 cells were infected with H5pg4100 (WT), H5pm4250 (UBM2) or H5pm4251 (UBM5) (m.o.i. 10) and fixed 48 h p.i. with 4% PFA. The immunodetection was carried out with the primary antibodies α-DBP (B6-8) and α-USP7 (3D8) as well as the secondary antibodies α- mouse-Texas red coupled and α-rat-Alexa488 coupled. The position of the cell nucleus was detected using the dye DAPI. Representative DBP and USP7 localization patterns of an average of 70 analyzed cells are shown (magnification x1000).

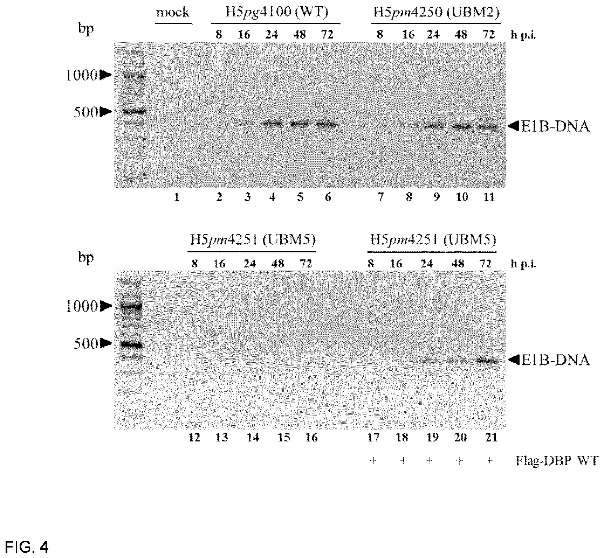

Figure 4 shows that the viral mutant UBM5 has a defect in the viral DNA synthesis. H1299 cells were infected with H5pg4100 (WT), H5pm4250 (UBM2) or H5pm4251 (UBM5) (m.o.i. 10) or transfected with 5 µg of the plasmid construct Flag-DBP WT followed by an UBM5 infection 24 h p.t. (m.o.i. 10). The cells were harvested 8-72 h p.i.. The viral DNA from the prepared lysates served as a template for a PCR with gene-specific oligonucleotides so that a PCR product of 389 bp from the HAdV-C5 E1B gene could be amplified and visualized on an agarose gel.

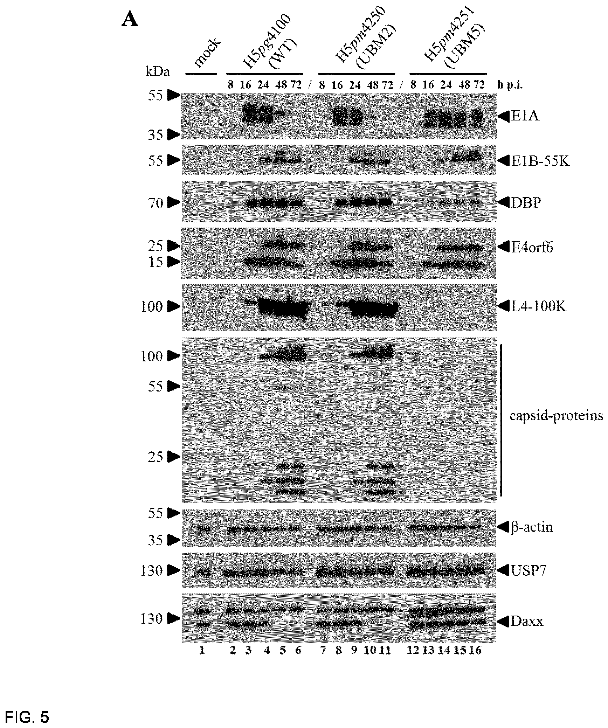

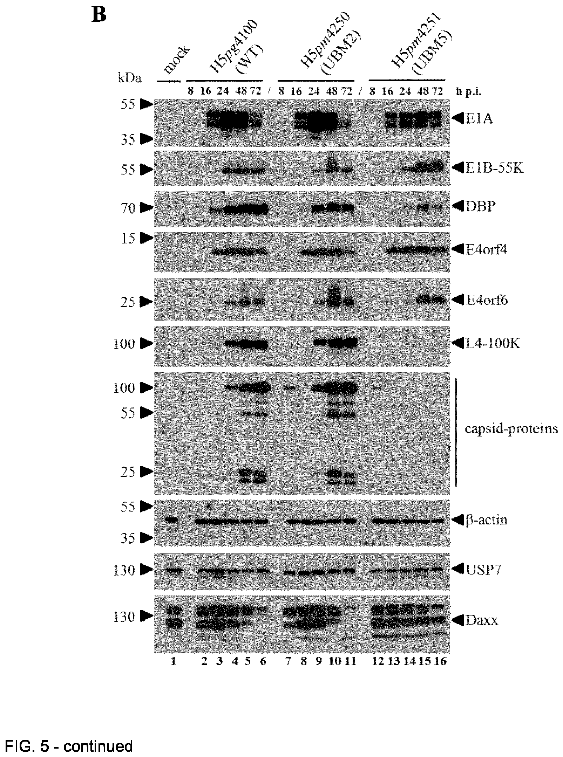

Figure 5 shows altered equilibrium quantities of viral and cellular proteins after UBM5 infection compared to the wild-type. H1299 cells (A) or HCT116 cells (B) were infected with H5pg4100 (WT), H5pm4250 (UBM2) or H5pm4251 (UBM5) at an m.o.i. of 20, harvested 8-72 h p.i. and lysed. The total protein cell extracts were separated by SDS-PAGE, and the proteins were visualized by immunodetection. Equilibrium quantities of specific proteins were detected with the antibodies M73 (α-E1A), 2A6 (α-E1B-55K), B6-8 (α-DBP), α-E4orf4, RSA3 (α-E4orf6), 6B10 (α-L4-100K), L133 (a-capsid proteins), α-β actin, 3D8 (α-USP7), α-Daxx and α-PML. The molecular weight in kDa is indicated on the left and the detected proteins on the right side of the figure.

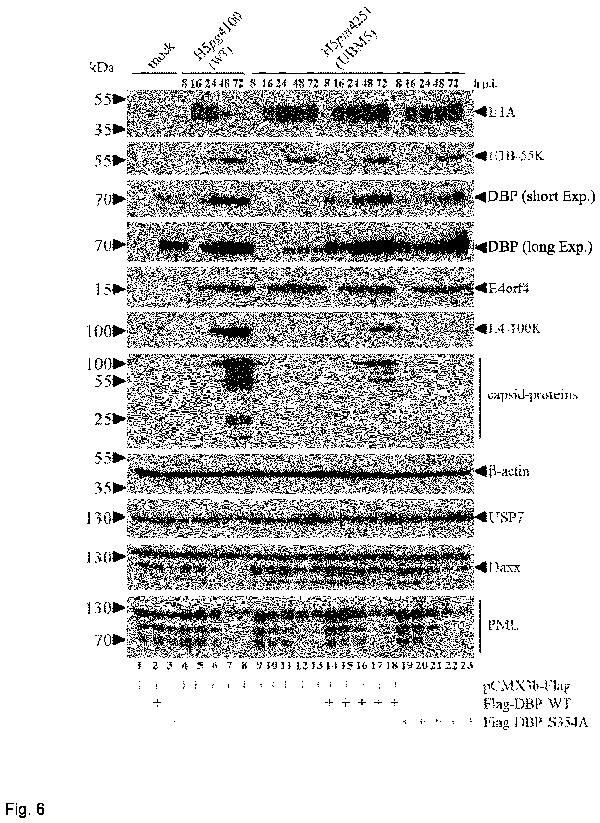

Figure 6 shows restoration of expression of late viral proteins in the viral mutant UBM5. H1299 cells were transfected with 5 µg of the plasmid construct flag-DBP WT or 10 µg of the plasmid construct flag-DBP S354A. In order to transfect the cells with the same amount of plasmid DNA in the different samples, the expression vector pCMX3b-flag was co-transfected if necessary. the transfected cells were infected with H5pg4100 (WT) or H5pm4251 (UBM5) (m.o.i. 10) 24 h p.t., harvested 8-72 h p.i. and lysed. The total protein cell extracts were separated by SDS-PAGE and the proteins were visualized by immunodetection. Equilibrium amounts of specific proteins were detected with the antibodies M73 (α-E1A), 2A6 (α-E1B-55K), B6-8 (a-DBP), α-E4orf4, RSA3 (α-E4orf6), 6B10 (α-L4-100K), L133 (α-capsid proteins), α-β actin, 3D8 (α-USP7), α-Daxx and α-PML.

### EXAMPLES

The present invention is further illustrated by the following examples, which in no way should be construed as limiting. The entire contents of all of the references (including literature references, issued patents, published patent applications, and co pending patent applications) cited throughout this application are hereby expressly incorporated by reference. The following materials and methods were used for performing the experiments described herein.

### Cell lines and culture conditions

H1299 (Mitsudomi et al., 1992), HCT116 (Brattain et al., 1981), HEK-293 (Graham et al., 1977) and 2E2 cells (Catalucci, 2005) were grown in Dulbecco's modified Eagle's Medium (DMEM) supplemented with 5 to 10% fetal bovine serum (FBS), 100 U/ml penicillin and 100 µg/ml streptomycin in a 5% CO₂ atmosphere at 37°C. For 2E2 cells, the medium was additionally supplemented with 90 µg/ml of hygromycin B and 250 µg/ml of geneticin (G418). In 2E2 cells, expression of the E2-gene region was induced by 1 µg/ml doxycycline.

### Plasmids and transient transfections

Both, pcDNA3 (Invitrogen) and pCMX3b encoding the cytomegalovirus (CMV) immediate-early promoter were used in the present study. HAdV-DBPs expressed from pCMX3b-based plasmids are flag tagged (Terzic, 2014). Human USP7 (tagged with myc; Zapata et al., 2001) was expressed from pcDNA3 based plasmid. Flag-DBP mutants were derived through nucleotide exchanges by site-directed mutagenesis using the following oligonucleotides:
E2A-UBM2 fwd primer
   5'-CCAGCCCGCGGCCATCGACCGCGGCGGCGGATTTGGCC-3',
E2A-UBM2 rev primer
   5'-GGCCAAATCCGCCGCCGCGGTCGATGGCCGCGGGCTGG-3';
E2A-UBM5 fwd primer
   5'-CCAA TCAGTTTTCCGGCAAGGCTTGCGGCATGTTCTTCTC-3',
E2A-UBM5 rev primer
   5'-GAGAAGAACATGCCGCAAGCCTTGCCGGAAAACTGATTGG-3'.

Transient transfection of subconfluent cells was performed with linear polyethylenimine (PEI; 25 kDa; Polysciences). The transfection solution was prepared by incubating a mixture of DNA, PEI and DMEM in a ratio of 1:6:60 (DNA:PEI:DMEM) for 10 min at room temperature (RT). Prior to transfection, the culture medium was replaced by DMEM without FBS and antibiotics. Transfection solution was added to the cells and incubated for 4 h in a 5% CO₂ atmosphere at 37°C before replacement of the medium with DMEM supplemented with 10% FCS, 100 U of penicillin and 100 µg of streptomycin per ml.

### Viruses and infections

The following viruses were used in the present study: H5pg4100 (wt), H5pm4250 (UBM2) and H5pm4251 (UBM5). H5pg4100 is an HAdV-C5-derived virus with deletions in the E3-coding region (Kindsmüller et al., 2007) and served as wild type virus. A nucleotide exchange in the DBP open reading frame of H5pm4250 or H5pm4251 resulted in a serine to alanine substitution at position 76 or 354, respectively. Mutagenesis of the DBP gene via site-directed mutagenesis PCR (Groitl & Dobner, 2007) was carried out with the following two primers for UBM2:
3151 - 5'-CCA GCC CGC GGC CAT CGA CCG CGG CGG CGG ATT TGG CC-3'
3152 - 5'-GGC CAA ATC CGC CGC CGC GGT CGA TGG CCG CGG GCT GG-3'
and the following two primers for UBM5:
3157 - 5'-CCA ATC AGT TTT CCG GCA AGG CTT GCG GCA TGT TCT TCT C-3'
3158 - 5'-GAG AAG AAC ATG CCG CAA GCC TTG CCG GAA AAC TGA TTG G-3'.

The mutated DBP open reading frames were used to generate the virus mutants as described previously (Groitl & Dobner, 2007; Zeller, 2005; Koyuncu & Dobner, 2009). Sequence analyses of the whole adenoviral genome ensured that only the desired mutation was inserted into the genome of these virus mutants during the whole process. H5pg4100 (wt) was propagated in H1299, HEK-293, whereas H5pm4251 (UBM5) was first generated in 2E2 cells. All viruses were titrated in H1299 cells. To measure viral progeny of infected cells, the cells were harvested, released viral particles and viral titer were determined as described before (Kindsmüller et al., 2009). Infection of cells was carried out with virus dilutions in DMEM without additive at indicated multiplicities of infection (m.o.i.). Two hours post infection DMEM containing FCS, penicillin and streptomycin was added to the virus-containing medium (1:1). Cells were harvested at the indicated time points.

### Antibodies

Primary antibodies specific for adenoviral proteins used in the present study included anti-DBP mouse mAb B6-8, α-E1A mouse mAb M73, α-E1B-55K mouse mAb 2A6, α-E4orf4 rabbit pAb, α-E4orf6 mouse mAb RSA3, α-L4-100K rat mAb 6B10 and α-capsid proteins rabbit pAb L133 (Kindsmüller, 2007). Primary antibodies for the detection of cellular and ectopically expressed proteins included α-β-actin mouse mAb (Sigma-Aldrich), α-flag mouse mAb M2 (Sigma-Aldrich), α-Daxx rabbit pAb, α-PML rabbit pAb, and α-USP7 rat mAb 3D8. Secondary antibodies conjugated to horseradish peroxidase (HRP) for detection of proteins by immunoblotting were anti-mouse IgG, α-rabbit IgG and α-rat IgG (Jackson, ImmunoResearch).

### Protein analysis and immunoprecipitation

Cell pellets of transfected or infected cells were lysed in ice-cold radioimmunoprecipitation assay (RIPA) buffer (50 mM Tris-HCl (pH 7,6), 150 mM NaCl, 5 mM EDTA, 1% NP-40, 0,5% sodium deoxycholate and 0,1% sodium dodecyl sulfate (SDS)) with protease inhibitors (1% (vol/vol) PMSF (phenylmethylsulfonyl fluoride), 0,1% (vol/vol) aprotinin, 1 µg/ml leupeptin, 1 µg/ml pepstatin) added upon usage on ice for 30 min. Cell lysates were sonicated for 30 sec at 4 °C (output 0.6; 0.8 impulses/s; Branson Sonifier 450) and centrifuged subsequently to pellet the cell debris (11000 rpm, 3 min, 4 °C). Protein concentration was determined photometrically with Bradford Reagent (BioRad). Protein samples were separated by SDS-PAGE after boiling for 3 min at 95°C in Laemmli buffer (5x). Proteins were transferred to nitrocellulose blotting membranes (0.45 µm pore size) and visualized by immunoblotting (Western blot) as described previously (Freudenberger et al., 2017). Protein lysates were additionally used for immunoprecipitation analyses. To investigate protein-protein interaction, proteins were immunoprecipitated as described previously (Berscheminski et al., 2012) with the exception that the Ab-coupled protein A-sepharose or flag-M2-coupled agarose (Sigma-Aldrich) was rotated overnight at 4 °C with the precleared protein lysates.

### Analysis of viral DNA synthesis by PCR

Adenoviral DNA replication was determined by PCR. At the indicated time points, infected cells were harvested and lysed in RIPA buffer as described above. Then, 10 µg of total cell lysates were treated with Tween-20 (final concentration: 0.5%; Applichem) and proteinase K (final concentration: 100 µg/ml; Roche) together with nucleic acid-free water (Promega) to a final volume of 100 µl. The samples were incubated for 1 h at 55 °C prior to enzyme inactivation for 10 min at 100 °C. 24.5 µl of each sample were used as DNA matrices for a PCR with specific oligonucleotides to amplify a fragment of 389 bp (E1B-55K-gene of HAdV-C5) as described before (Ching et al., 2013). The PCR products were analyzed on 1% agarose gels containing ethidium bromide.

### Example 1: DBP-USP7 binding studies

In order to investigate the hypothesis that USP7 relocalization is mediated by DBP, a putative protein-protein interaction of DBP with transfected and endogenous USP7 was investigated in human H1299 and HCT116 cells. These two cell lines represent different infection targets of adenoviruses (Brattain et al., 1981; Mitsudomi et al., 1992). First, immunoprecipitation of DBP with wt-virus (H5pg4100) infected H1299 and HCT116 cells and subsequent staining of USP7 showed an interaction between both (data not shown). To test whether this interaction is independent of other viral proteins, H1299 and HCT116 cells were transfected with a plasmid encoding flag-DBP and myc-USP7, respectively. In H1299 cells both endogenous and overexpressed USP7 could be coprecipitated with DBP (Figure 1A) whereas in HCT cells only overexpressed USP7 was coprecipitated with DBP (Figure 1B). The infection analyses confirmed USP7-DBP interaction during the infection cycle. Additionally, the transfection analysis with the lack of further viral proteins shows that the aforementioned interaction is independent from other viral proteins.

To characterize the interaction in more detail, the DBP binding domain of USP7 was investigated. USP7 consists of the N-terminal TRAF (tumor necrosis factor receptor (TNFR)-associated factor)-like domain, central catalytic domain and five C-terminal UBL (ubiquitin-like)-domains (Holowaty et al., 2003a; Zapata et al., 2001; Faesen et al., 2011). GST fusion proteins corresponding to these USP7-domains were used and their interaction with DBP was evaluated by GST pull-down experiments (data not shown). The first 215 residues of USP7, which correspond to the TRAF-like domain of USP7, precipitated strongly and specifically DBP from transfected and wt-virus (H5pg4100) infected cells (data not shown). Whereas binding with the other GST-fused USP7-fragments or GST alone is not detectable (data not shown). This result is in accordance with already published USP7-interaction partners (e.g. p53, MDM2), which had also been shown to interact with the TRAF-like domain of USP7 (Sheng et al., 2006; Holowaty et al., 2003a).

### Example 2: Generation of DBP variants

Several USP7-interaction partners like p53, MDM2 and EBNA1 encode the consensus motifs "X-Gly-X-Ser" or "Pro/Ala-X-X-Ser" which provide for USP7 binding (Sheng et al., 2006). Therefore, the amino acid sequence of DBP was analyzed for USP7-binding motifs (UBM) and five potential USP7-binding sites were identified (31-Pro-Ser-Pro-Ser-36, 72-Pro-Ser-Thr-Ser-77, 118-Val-Gly-Phe-Ser-123, 175-Pro-Iso-Val-Ser-180, 350-Ser-Gly-Lys-Ser-355). In order to analyze the putative USP7-binding sites in DBP, the last serine of each motif was substituted (from Ser to Ala) and the interaction of these DBP variants with USP7 was investigated in immunoprecipitation experiments. The expression levels of the DBP variants were comparable except for the mutant DBP-S354A whose protein level was reduced (data not shown). USP7 interaction could be proven with almost all DBP variants although binding to the mutant DBP-S354A was decreased. Strikingly, the interaction of USP7 with mutant DBP-S76A is abolished.

To further verify the abolished interaction between USP7 and the mutant DBP-S76A, GST pull-down assays were performed. For this purpose, the GST-fused TRAF-like domain of USP7 was used and lysates of H1299 cells were transfected with the DBP-variants. Consistent with the immunoprecipitation analyses shown before, DBP-variants were pulled-down with the GST-tagged TRAF-like domain of USP7 (data not shown) except of the mutant DBP-S76A. Although the protein levels of DBP-S354A were decreased in comparison to the other DBP-variants, the amount of pulled-down DBP-S354A was comparable to DBP-wt (data not shown). These results indicate that the TRAF-like domain of USP7 interacts with the "Pro-Ser-Thr-Ser" motif of DBP.

### Example 3: Generation of virus mutants

To clarify the role of the DBP-variants in virus infection, virus mutants were prepared. DBP-variants with a different phenotype in transfection compared to wild-type DBP (DBP-wt) were selected. Hence, the DBP amino acid exchange mutant S76A (UBM2), which is deficient in USP7 binding, and the mutant S354A (UBM5), which exerts reduced protein levels, were selected. USP7-binding of the newly generated DBP-mutants in infection was investigated via immunoprecipitation analyses. Consistent with the transfection experiments, DBP-S76A of UBM2 did not bind to USP7 in infection (Figure 2). Despite of decreased protein levels of DBP-S354A, the levels of coprecipitated USP7 with the UBM5-DBP are comparable to the DBP-wt (Figure 2). These data demonstrate that the "PSTS" motif (aa 73-76) in the N-terminal domain of DBP is crucial for USP7-binding. Furthermore, these data showed that the amino acid exchange of aa 354 in the C-terminal domain of DBP influences the expression or stability of this protein.

### Example 4: Functional studies

In order to investigate the implication of DBP binding to the intranuclear relocalization of USP7 into viral replication centers, H1299 cells were infected with wt-, UBM2- or UBM5-virus and analyzed via immunofluorescence analysis. Non-infected cells showed a diffuse nuclear distribution of USP7, while it was relocalized to viral replication centers in wt-infected cells independent of the investigated time point. During the infection progress, different morphologies of the replication centers could be observed, nevertheless USP7 and DBP always colocalized in wt-infected cells.

In UBM2-infected cells DBP-S76A localized comparable to DBP-wt, but the relocalization of USP7 into viral replication centers was completely abolished at 8, 16 and 24 h p.i. in 100% of the infected cells (data not shown). A partial relocalization of USP7 into ring-like or rosette-shaped replication centers was observed 48 h p.i. in 55% of the UBM2-infected cells (Figure 3). However, USP7 was still diffusely distributed within the nucleus in 45% of the UBM2-infected cells. Therefore, it was concluded that the binding of USP7 by DBP is necessary for relocalization of USP7 during infection, as DBP-S76A from the UBM2-virus is unable to bind USP7.

In UBM5-infected cells both DBP and USP7 localized diffusely in the nucleus at all investigated time points and in all evaluated cells. (data not shown). Thus, the UBM5-virus is completely defective in the establishment of replication centers. To finally show that the amino acid exchange S354A is responsible for the affected replication centers formation, a plasmid encoding DBP-wt was transfected before UBM5 infection (data not shown). This led to detectable replication centers and a relocalization of USP7 in UBM5-infected cells.

In summary, it was demonstrated that the amino acid exchange S76A in DBP prevents the relocalization of USP7, especially at early time points of infection, whereas the amino acid exchange S354A in DBP abolishes the establishment of viral replication centers.

### Example 5: Analysis of DNA replication and virus progeny production

Since immunofluorescence analyses showed impaired USP7 relocalization in UBM2-virus infected cells and failure to establish replication centers for the UBM5-virus, the consequences of these phenotypes on viral DNA replication were further analyzed. To investigate this, H1299 cells were infected with wt-, UBM2- or UBM5-virus, and the isolated viral DNA was analyzed by standard PCR with E1B-gene-specific oligonucleotides. PCR products could be detected starting 16 h p.i. from the wt-virus and the UBM2-virus infected cell extracts (Figure 4). As expected, PCR products were absent in the UBM5-virus infected cell extracts (Figure 4). To narrow down this effect on the mutation of DBP, H1299 cells were transfected with a plasmid encoding DBP-wt before infecting with UBM5-virus. As a consequence, viral DNA could be detected in the UBM5-virus infected cells starting 24 h p.i.

Therefore, it can be concluded that the DNA replication of the UBM2-virus is intact, despite the affected recruitment of USP7 into replication centers. However, the UBM5-virus has a severe defect in viral DNA replication consistent with the defective replication centers formation in UBM5-infected cells. To verify this data with a quantitative and more sensitive method, the viral DNA replication was investigated with real time qPCRs. It was found that the amount of incoming viral DNA at 1 h p.i. was comparable for wt, UBM2 and UBM5 infected cells. During the course of infection however, the UBM2-virus showed a slightly increased DNA synthesis compared to wt-virus. Especially 48 h p.i. about 20% more viral DNA was detected in the UBM2-infected cells. In contrast, the amount of viral DNA did not increase in the UBM5-infected cells during the infection cycle. The amount of detected DNA corresponded to the amount of incoming viral DNA at 1 h p.i. In line with the results shown before, the UBM5-virus is affected severely in replicating viral DNA, whereas the UBM2-virus seems to replicate slightly more efficient than the wt-virus.

Subsequently, H1299 cells were infected with wt-, UBM2- or UBM5-virus to determine the progeny production by titration analyses/fluorescence focus identification assay. Consistent with the replication defect, virus progeny production is completely abolished in UBM5-virus infected cells. As for the UBM2-virus, slightly increased progeny production of 28% 24 h p.i. and 17% 48 h p.i. was observed compared to wt-virus. This is in agreement with the observed increase in DNA synthesis for the UBM2-virus compared to wt-virus. In light of this, it can be concluded that the amino acid exchange S76A in DBP has a mild effect on viral progeny production, while the amino acid exchange S354A in DBP prevents virus progeny production as a result of defective DNA replication.

### Example 6: Analysis of late viral protein expression

It is well known that the onset of viral DNA replication is crucial for the expression of late viral genes and thereby induces the transition from early to late phase of infection (Seth, 1999). As the virus mutants showed altered or defective viral DNA synthesis, we investigated their expression of early and late viral proteins. H1299 cells were infected with wt-, UBM2- or UBM5-virus, and cell lysates were investigated with Western Blot-analyses. The early and late protein levels of UBM2-virus compared to wt-virus infected cells were similar (Figure 5A, compare lane 2-6 with lane 7-11). However, UBM5-virus infected cells showed a few differences in early protein expression and huge differences in late protein expression in comparison to wt-virus infected cells (Figure 5A, compare lane 2-6 with lane 12-16). The first protein expressed during infection is the early protein E1A, which was expressed during the entire infection cycle in UBM5-infected cells in contrast to wt-virus infected cells in which the E1A levels peaked at 16 h p.i. and decreased over time. As previously observed, the UBM5-virus showed decreased DBP protein levels during the infection cycle compared to the wt-virus. Strikingly, UBM5-virus infection is entirely defective in late protein expression, which is represented by the absence of late protein L4-100K and capsid proteins expression (Figure 5A, lane 12-16). Thus, as a consequence of defective DNA replication of the UBM5-virus is incapable to induce late viral protein expression. Almost identical results were obtained when HCT116 cells were infected the wild-type virus (Figure 5B, lanes 2-6) and the UBM2 and UBM5 mutant viruses (Figure 5B, lanes 7-11 and 12-16, respectively).

To verify that the observed defect in late protein expression is due to the amino acid exchange in DBP, the experiment was repeated with UBM5-virus infected cells additionally transfected with a plasmid encoding wild-type DBP. The results showed that late viral protein expression could be rescued in the UBM5-virus infected cells after transfection with DBP-wt proving that the UBM5 mutation induces the defect (Figure 6). Furthermore, this experiment was performed to exclude that a threshold of DBP is needed for the formation of replication centers and therefore the transition from early to late phase of infection. Hence, UBM5-virus infected H1299 cells were transfected with a plasmid encoding the mutant DBP-S354A to adjust the expression level of DBP to wt-infection. Although, the UBM5-DBP level was similar to DBP-wt, late protein expression was still defective after UBM5-virus infection excluding the possibility that the reduced UBM5-DBP level causes the defect in late protein expression. In summary, the amino acid exchange S354A in DBP abolishes the transition into the late phase of infection and therefore affects late protein expression during UBM5-virus infection.

### LITERATURE

Abe S., Miyamura K., Oba T., Terakura S., Kasai M., Kitaori K., Sasaki T., Kodera Y. (2003); Bone marrow transplantation, 32, 1107-1108.
Ali A., RAJA R., Farooqui S.R., Ahmad S., Banerjea A.C. (2017); Biochemical Journal, 474, BCJ20160304.
Ampuero J.S., Ocaña V., Gómez J., Gamero M.E., Garcia J., Halsey E.S., Laguna-Torres V.A. (2012); PLoS ONE, 7.
Berscheminski J, Groitl P, Dobner T, Wimmer P & Schreiner S (2012); Journal of Virology 87, 965-977.
van Breukelen B., Brenkman A.B., Holthuizen P.E., van der Vliet P.C. (2003); Society, 77, 915-922.
van Breukelen B., Kanellopoulos P.N., Tucker P.A., van der Vliet P.C. (2000); Journal of Biological Chemistry, 275, 40897-40903.
Brattain M.G., Fine W.D., Khaled F.M., Thompson J., Brattain D.E. (1981); Cancer research, 41, 1751-6.
Canning M., Boutell C., Parkinson J., Everett R.D. (2004); Journal of Biological Chemistry, 279,38160-38168.
Carrigan D.R. (1997); American journal of Medicine, 102, 71-74.
Catalucci D., Sporeno E., Cirillo A., Ciliberto G., Nicosia A., Colloca S. (2005); Journal of virology, 79, 6400-6409.
Ching W., Koyuncu E., Singh S., Arbelo-Roman C., Hartl B., Kremmer E., Speiseder T., Meier C., Dobner T. (2013); PLoS Pathogens, 9.
Chow L.T., Lewis J.B., Broker T.R. (1980); Cold Spring Harbor symposia on quantitative biology, 44 Pt 1, 401-414.
Condezo G.N., San Martin C. (2017); PLoS Pathogens, 13, e1006320.
Dar A., Shibata E., Dutta A. (2013); Molecular and Cellular Biology, 33, 3309-3320.
Dekker J., Kanellopoulos P.N., Loonstra A.K., van Oosterhout J.A.W.M., Leonard K., Tucker P.A., van der Vliet P.C. (1997); EMBO Journal, 16, 1455-1463.
Faesen A.C., Dirac A.M.G., Shanmugham A., Ovaa H., Perrakis A., Sixma T.K. (2011); Molecular Cell, 44, 147-159.
Faustrup H., Bekker-Jensen S., Bartek J., Lukas J., Mailand N. (2009); Journal of Cell Biology, 184, 13-19.
Felle M., Joppien S., Németh A., Diermeier S., Thalhammer V., Dobner T., Kremmer E., Kappler R., Langst G. (2011); Nucleic Acids Research, 39, 8355-8365.
Flint S.J., Sharp P.A. (1976); Journal of Molecular Biology, 106, 749-771.
Freudenberger N, Meyer T, Groitl P, Dobner T & Schreiner S (2017); Journal of Virology, JVI.01451-17-20.
Graham F.L., Smiley J., Russell W.C., Nairn R. (1977); Journal of General Virology, 36, 59-72.
Groitl P., Dobner T. (2007); Methods Mol.Med., 130, 29-39.
Hidalgo P., Anzures L., Hernandez-Mendoza A., Guerrero A., Wood C.D., Valdés M., Dobner T., Gonzalez R.A. (2016); Journal of Virology, JVI.00033-16.
Holowaty M.N., Zeghouf M., Wu H., Tellam J., Athanasopoulos V., Greenblatt J., Frappier L. (2003); Journal of Biological Chemistry, 278, 29987-29994.
Holowaty MN, Sheng Y, Nguyen T, Arrowsmith C & Frappier L (2003); J. Biol. Chem. 278, 47753-47761.
van der Horst A., Vries-smits A.M.M. De, Brenkman A.B., van Triest M.H., van den Broek N., Colland F., Maurice M.M., Burgering B.M.T., van der Horst A., de Vries-Smits A.M.M., Brenkman A.B., van Triest M.H., van den Broek N., Colland F., Maurice M.M., Burgering B.M.T. (2006); Nature cell biology, 8, 1064-73.
Jäger W., Santag S., Weidner-Glunde M., Gellermann E., Kati S., Pietrek M., Viejo-Borbolla A., Schulz T.F. (2012); Journal of Virology, 86, 6745-6757.
Khoronenkova S. V., Dianov G.L. (2013); Nucleic Acids Research, 41, 1750-1756.
Kindsmüller K., Groitl P., Härtl B., Blanchette P., Hauber J., Dobner T. (2007); Proc. Natl. Acad. Sci. USA, 104, 6684-6689. Kolawole O.M., Oladosu T.O., Abdulkarim A.A., Okoh A.I. (2014); BMC research notes, 7, 870.
Kindsmuller K, Schreiner S, Leinenkugel F, Groitl P, Kremmer E & Dobner T (2009); Journal of Virology 83, 9045-9056.
Koyuncu O.Ö., Dobner T. (2009); Journal of virology, 83, 4778-4790.
Li M., Brooks C.L., Kon N., Gu W. (2004); Molecular Cell, 13, 879-886.
Li M., Chen D., Shiloh A., Luo J., Nikolaev A.Y., Qin J., Gu W. (2002); Nature, 416, 648-653.
Lin K.H., Lin Y.C., Chen H.L., Ke G.M., Chiang C.J., Hwang K.P., Chu P.Y., Lin J.H., Liu D.P., Chen H.Y. (2004); Journal of Medical Virology, 73, 274-279.
Lindenbaum J.O., Field J., Hurwitz J. (1986); Journal of Biological Chemistry, 261, 10218-10227.
Lion T., Kosulin K., Landlinger C., Rauch M., Preuner S., Jugovic D., Pötschger U., Lawitschka A., Peters C., Fritsch G., Matthes-Martin S. (2010); Leukemia, 24, 706-714.
Mahy B.W.J., van Regenmortel M.H.V. (2010) Desk encyclopedia of human and medical virology.
Mitchell L.S., Taylor B., Reimels W., Barrett F.F., Devincenzo J.P. (2000); The Pediatric Infectious Disease Journal, 19, 996-1000.
Mitsudomi T., Steinberg S.M., Nau M.M., Carbone D., D'Amico D., Bodner S., Oie H.K., Linnoila R.I., Mulshine J.L., Minna J.D., et al. (1992); Oncogene, 7, 171-180.
Monaghan A., Webster A., Hay R.T. (1994); Nucleic Acids Research, 22, 742-748.
Pombo A., Ferreira J., Bridge E., Carmo-Fonseca M. (1994); The EMBO journal, 13, 5075-85.
Salsman J., Jagannathan M., Paladino P., Chan P.-K., Dellaire G., Raught B., Frappier L. (2012); Journal of virology, 86, 806-20.
Seth P. (1999) Adenoviruses : Basic Biology to Gene Therapy.
Sheng Y., Saridakis V., Sarkari F., Duan S., Wu T., Arrowsmith C.H., Frappier L. (2006); Nature structural & molecular biology, 13, 285-291.
Song M.S., Salmena L., Carracedo A., Egia A., Lo-Coco F., Teruya-Feldstein J., Pandolfi P.P. (2008); Nature, 455, 813-7.
Terzic M. (2014); Role of E2A/DBP SUMOylation during productive infection with Human Adenovirus Type 5. University of Belgrade.
van der Vliet P.C., Levine A.J. (1973); Nature, 246, 170-174.
Voelkerding K., Klessig D.F. (1986); Journal of virology, 60, 353-62.
Zapata J.M.J.M., Pawlowski K., Haas E., Ware C.F.C.F., Godzik A., Reed J.C.J.C. (2001); Journal of Biological Chemistry, 276, 24242-24252.
Zijderveld D.C., van der Vliet P.C. (1994); Journal of Virology, 68, 1158-1164.

## Claims

1. Adenoviral DNA-binding protein (DBP) comprising a mutation in the sequence motif NH₂-Ser-[Gly/Ser/Ala]-[Lys/Arg]-Ser-COOH which inhibits adenoviral DNA replication in a cell infected with a virus expressing said protein.

2. Adenoviral DNA-binding protein (DBP) of claim 1, wherein said mutation is an amino acid substitution or deletion of the Ser residue located at the COOH terminus of the sequence motif.

3. Adenoviral DNA-binding protein (DBP) of claim 2, wherein said mutation is an amino acid substitution from Ser to Ala.

4. Adenoviral DNA-binding protein (DBP) of any of claims 1-3, wherein said protein comprises the amino acid sequence of SEQ ID NO:2 or an amino acid sequence having at least 90% identity thereto.

5. Nucleotide sequence encoding the adenoviral DNA-binding protein (DBP) of any of claims 1-4.

6. Plasmid comprising the nucleotide sequence of claim 5.

7. Adenovirus or recombinant adenoviral vector comprising the adenoviral DNA-binding protein (DBP) of any of claims 1-4 or the nucleotide sequence of claim 5.

8. Adenovirus or recombinant adenoviral vector of claim 7, wherein said adenovirus or adenoviral vector belongs to a type selected from the group of HAdV types 1, 2, 3, 4, 5, 6, 7, 31, 40, and 41.

9. Adenoviral DNA-binding protein (DBP) of any of claims 1-4, nucleotide sequence of claim 5, plasmid of claim 6, or adenovirus or recombinant adenoviral vector of any of claims 7-8 for use in medicine.

10. Adenoviral DNA-binding protein (DBP) of any of claims 1-4, nucleotide sequence of claim 5, plasmid of claim 6, or adenovirus or recombinant adenoviral vector of any of claims 7-8 for use in a method of treating an adenovirus infection.

11. Adenoviral DNA-binding protein (DBP) of any of claims 1-4, nucleotide sequence of claim 5, plasmid of claim 6, or adenovirus or recombinant adenoviral vector of any of claims 7-8 for use in a method of vaccinating a subject.

12. Adenoviral DNA-binding protein (DBP), nucleotide sequence, plasmid, or adenovirus or recombinant adenoviral vector for use in method of claim 11, wherein said subject is vaccinated against a disease, which is caused by an adenovirus and is selected from the group consisting of keratoconjunctivitis epidemica, acute respiratory diseases, pharyngoconjunctival fever, follicular conjuncttivitis, gastroenteritis, gastroenteritis with mesenteric lymphadenopathy, pneumonia, and pharyngitis.

13. Adenoviral DNA-binding protein (DBP) of any of claims 1-4, nucleotide sequence of claim 5, plasmid of claim 6, or adenovirus or recombinant adenoviral vector of any of claims 7-8 for use in a gene therapy method.

14. Cell comprising an adenoviral DNA-binding protein (DBP) of any of claims 1-4, a nucleotide sequence of claim 5, a plasmid of claim 6, or an adenovirus or recombinant adenoviral vector of any of claims 7-8.

15. Pharmaceutical composition or vaccine comprising an adenoviral DNA-binding protein (DBP) of any of claims 1-4, a nucleotide sequence of claim 5, a plasmid of claim 6, or an adenovirus or recombinant adenoviral vector of any of claims 7-8.

16. Use of an adenoviral DNA-binding protein (DBP) of any of claims 1-4, a nucleotide sequence of claim 5, a plasmid of claim 6, or an adenovirus or recombinant adenoviral vector of any of claims 7-8 for the preparation of a vaccine.


SEQUENCE LISTING

<110> Heinrich-Pette-Institut

<120> REPLICATION-DEFICIENT ADENOVIRUS

<130> P 109929

<160> 10

<170> BiSSAP 1.3.6

<210> 1
<211> 529
<212> PRT
<213> Adenoviridae

<220>
<223> Wild-type amino acid sequence of DBP of HAdV-5

<400> 1
   Met Ala Ser Arg Glu Glu Glu Gln Arg Glu Thr Thr Pro Glu Arg Gly
   1               5                   10                  15
   Arg Gly Ala Ala Arg Arg Pro Pro Thr Met Glu Asp Val Ser Ser Pro
               20                  25                  30
   Ser Pro Ser Pro Pro Pro Pro Arg Ala Pro Pro Lys Lys Arg Met Arg
           35                  40                  45
   Arg Arg Ile Glu Ser Glu Asp Glu Glu Asp Ser Ser Gln Asp Ala Leu
       50                  55                  60
   Val Pro Arg Thr Pro Ser Pro Arg Pro Ser Thr Ser Ala Ala Asp Leu
   65                  70                  75                  80
   Ala Ile Ala Pro Lys Lys Lys Lys Lys Arg Pro Ser Pro Lys Pro Glu
                   85                  90                  95
   Arg Pro Pro Ser Pro Glu Val Ile Val Asp Ser Glu Glu Glu Arg Glu
               100                 105                 110
   Asp Val Ala Leu Gln Met Val Gly Phe Ser Asn Pro Pro Val Leu Ile
           115                 120                 125
   Lys His Gly Lys Gly Gly Lys Arg Thr Val Arg Arg Leu Asn Glu Asp
       130                 135                 140
   Asp Pro Val Ala Arg Gly Met Arg Thr Gln Glu Glu Glu Glu Glu Pro
   145                 150                 155                 160
   Ser Glu Ala Glu Ser Glu Ile Thr Val Met Asn Pro Leu Ser Val Pro
                   165                 170                 175
   Ile Val Ser Ala Trp Glu Lys Gly Met Glu Ala Ala Arg Ala Leu Met
               180                 185                 190
   Asp Lys Tyr His Val Asp Asn Asp Leu Lys Ala Asn Phe Lys Leu Leu
           195                 200                 205
   Pro Asp Gln Val Glu Ala Leu Ala Ala Val Cys Lys Thr Trp Leu Asn
       210                 215                 220
   Glu Glu His Arg Gly Leu Gln Leu Thr Phe Thr Ser Asn Lys Thr Phe
   225                 230                 235                 240
   Val Thr Met Met Gly Arg Phe Leu Gln Ala Tyr Leu Gln Ser Phe Ala
                   245                 250                 255
   Glu Val Thr Tyr Lys His His Glu Pro Thr Gly Cys Ala Leu Trp Leu
               260                 265                 270
   His Arg Cys Ala Glu Ile Glu Gly Glu Leu Lys Cys Leu His Gly Ser
           275                 280                 285
   Ile Met Ile Asn Lys Glu His Val Ile Glu Met Asp Val Thr Ser Glu
       290                 295                 300
   Asn Gly Gln Arg Ala Leu Lys Glu Gln Ser Ser Lys Ala Lys Ile Val
   305                 310                 315                 320
   Lys Asn Arg Trp Gly Arg Asn Val Val Gln Ile Ser Asn Thr Asp Ala
   325 330 335
   Arg Cys Cys Val His Asp Ala Ala Cys Pro Ala Asn Gln Phe Ser Gly
   340 345 350
   Lys Ser Cys Gly Met Phe Phe Ser Glu Gly Ala Lys Ala Gln Val Ala
   355 360 365
   Phe Lys Gln Ile Lys Ala Phe Met Gln Ala Leu Tyr Pro Asn Ala Gln
   370 375 380
   Thr Gly His Gly His Leu Leu Met Pro Leu Arg Cys Glu Cys Asn Ser
   385 390 395 400
   Lys Pro Gly His Ala Pro Phe Leu Gly Arg Gln Leu Pro Lys Leu Thr
   405 410 415
   Pro Phe Ala Leu Ser Asn Ala Glu Asp Leu Asp Ala Asp Leu Ile Ser
   420 425 430
   Asp Lys Ser Val Leu Ala Ser Val His His Pro Ala Leu Ile Val Phe
   435 440 445
   Gln Cys Cys Asn Pro Val Tyr Arg Asn Ser Arg Ala Gln Gly Gly Gly
   450 455 460
   Pro Asn Cys Asp Phe Lys Ile Ser Ala Pro Asp Leu Leu Asn Ala Leu
   465 470 475 480
   Val Met Val Arg Ser Leu Trp Ser Glu Asn Phe Thr Glu Leu Pro Arg
   485 490 495
   Met Val Val Pro Glu Phe Lys Trp Ser Thr Lys His Gln Tyr Arg Asn
   500 505 510
   Val Ser Leu Pro Val Ala His Ser Asp Ala Arg Gln Asn Pro Phe Asp
   515 520 525
   Phe

<210> 2
<211> 529
<212> PRT
<213> Adenoviridae

<220>
<223> Mutated amino acid sequence of DBP of HAdV-5

<400> 2
Met Ala Ser Arg Glu Glu Glu Gln Arg Glu Thr Thr Pro Glu Arg Gly
1               5                   10                  15
Arg Gly Ala Ala Arg Arg Pro Pro Thr Met Glu Asp Val Ser Ser Pro
            20                  25                  30
Ser Pro Ser Pro Pro Pro Pro Arg Ala Pro Pro Lys Lys Arg Met Arg
        35                  40                  45
Arg Arg Ile Glu Ser Glu Asp Glu Glu Asp Ser Ser Gln Asp Ala Leu
    50                  55                  60
Val Pro Arg Thr Pro Ser Pro Arg Pro Ser Thr Ser Ala Ala Asp Leu
65                  70                  75                  80
Ala Ile Ala Pro Lys Lys Lys Lys Lys Arg Pro Ser Pro Lys Pro Glu
                85                  90                  95
Arg Pro Pro Ser Pro Glu Val Ile Val Asp Ser Glu Glu Glu Arg Glu
            100                 105                 110
Asp Val Ala Leu Gln Met Val Gly Phe Ser Asn Pro Pro Val Leu Ile
        115                 120                 125
Lys His Gly Lys Gly Gly Lys Arg Thr Val Arg Arg Leu Asn Glu Asp
    130                 135                 140
Asp Pro Val Ala Arg Gly Met Arg Thr Gln Glu Glu Glu Glu Glu Pro
145                 150                 155                 160
Ser Glu Ala Glu Ser Glu Ile Thr Val Met Asn Pro Leu Ser Val Pro
                165                 170                 175
Ile Val Ser Ala Trp Glu Lys Gly Met Glu Ala Ala Arg Ala Leu Met
            180                 185                 190
Asp Lys Tyr His Val Asp Asn Asp Leu Lys Ala Asn Phe Lys Leu Leu

195                 200                 205
Pro Asp Gln Val Glu Ala Leu Ala Ala Val Cys Lys Thr Trp Leu Asn
    210                 215                 220
Glu Glu His Arg Gly Leu Gln Leu Thr Phe Thr Ser Asn Lys Thr Phe
225                 230                 235                 240
Val Thr Met Met Gly Arg Phe Leu Gln Ala Tyr Leu Gln Ser Phe Ala
                245                 250                 255
Glu Val Thr Tyr Lys His His Glu Pro Thr Gly Cys Ala Leu Trp Leu
            260                 265                 270
His Arg Cys Ala Glu Ile Glu Gly Glu Leu Lys Cys Leu His Gly Ser
        275                 280                 285
Ile Met Ile Asn Lys Glu His Val Ile Glu Met Asp Val Thr Ser Glu
    290                 295                 300
Asn Gly Gln Arg Ala Leu Lys Glu Gln Ser Ser Lys Ala Lys Ile Val
305                 310                 315                 320
Lys Asn Arg Trp Gly Arg Asn Val Val Gln Ile Ser Asn Thr Asp Ala
                325                 330                 335
Arg Cys Cys Val His Asp Ala Ala Cys Pro Ala Asn Gln Phe Ser Gly
            340                 345                 350
Lys Ala Cys Gly Met Phe Phe Ser Glu Gly Ala Lys Ala Gln Val Ala
        355                 360                 365
Phe Lys Gln Ile Lys Ala Phe Met Gln Ala Leu Tyr Pro Asn Ala Gln
    370                 375                 380
Thr Gly His Gly His Leu Leu Met Pro Leu Arg Cys Glu Cys Asn Ser
385                 390                 395                 400
Lys Pro Gly His Ala Pro Phe Leu Gly Arg Gln Leu Pro Lys Leu Thr
                405                 410                 415
Pro Phe Ala Leu Ser Asn Ala Glu Asp Leu Asp Ala Asp Leu Ile Ser
            420                 425                 430
Asp Lys Ser Val Leu Ala Ser Val His His Pro Ala Leu Ile Val Phe
        435                 440                 445
Gln Cys Cys Asn Pro Val Tyr Arg Asn Ser Arg Ala Gln Gly Gly Gly
    450                 455                 460
Pro Asn Cys Asp Phe Lys Ile Ser Ala Pro Asp Leu Leu Asn Ala Leu
465                 470                 475                 480
Val Met Val Arg Ser Leu Trp Ser Glu Asn Phe Thr Glu Leu Pro Arg
                485                 490                 495
Met Val Val Pro Glu Phe Lys Trp Ser Thr Lys His Gln Tyr Arg Asn
            500                 505                 510
Val Ser Leu Pro Val Ala His Ser Asp Ala Arg Gln Asn Pro Phe Asp
        515                 520                 525
Phe

<210> 3
<211> 38
<212> DNA
<213> Artificial Sequence

<220>
<223> oligonucleotide primer

<400> 3
ccagcccgcg gccatcgacc gcggcggcgg atttggcc                              38

<210> 4
<211> 38
<212> DNA
<213> Artificial Sequence

<220>
<223> oligonucleotide primer

<400> 4
ggccaaatcc gccgccgcgg tcgatggccg cgggctgg                                38


<210> 5
<211> 40
<212> DNA
<213> Artificial Sequence


<220>
<223> oligonucleotide primer

<400> 5
ccaatcagtt ttccggcaag gcttgcggca tgttcttctc                              40


<210> 6
<211> 40
<212> DNA
<213> Artificial Sequence


<220>
<223> oligonucleotide primer

<400> 6
gagaagaaca tgccgcaagc cttgccggaa aactgattgg                              40


<210> 7
<211> 38
<212> DNA
<213> Artificial Sequence


<220>
<223> oligonucleotide primer

<400> 7
ccagcccgcg gccatcgacc gcggcggcgg atttggcc                                38


<210> 8
<211> 38
<212> DNA
<213> Artificial Sequence


<220>
<223> oligonucleotide primer

<400> 8
ggccaaatcc gccgccgcgg tcgatggccg cgggctgg                                38


<210> 9
<211> 40
<212> DNA
<213> Artificial Sequence

<220>
<223> oligonucleotide primer

<400> 9
ccaatcagtt ttccggcaag gcttgcggca tgttcttctc                              40


<210> 10
<211> 40
<212> DNA
<213> Artificial Sequence


<220>
<223> oligonucleotide primer

<400> 10
gagaagaaca tgccgcaagc cttgccggaa aactgattgg                              40